(19) Europäisches Patentamt
European Patent Office
Office européen des brevets

(11) **EP 0 787 977 B1**

(12) **FASCICULE DE BREVET EUROPEEN**

(45) Date de publication et mention
de la délivrance du brevet:
**06.05.2004 Bulletin 2004/19**

(51) Int Cl.⁷: **A61B 8/06**, G01P 5/00,
G01F 1/66

(21) Numéro de dépôt: **97104304.7**

(22) Date de dépôt: **20.09.1993**

(54) **Sonde intracorporelle pour déterminer avec précision la vitesse d'un milieu liquide et, en particulier, le débit aortique**

Intracorporalen Sonde zur genauen Bestimmung der Geschwindigkeit einer Flüssigkeit, insbesondere des Durchflusses durch die Aorta

Intracorporal probe for precise determination of the velocity of a liquid, in particular of aortic flow

(84) Etats contractants désignés:
**AT BE CH DE DK ES FR GB GR IE IT LI LU NL PT SE**

(30) Priorité: **21.09.1992 FR 9211425**
**24.05.1993 US 64900**

(43) Date de publication de la demande:
**06.08.1997 Bulletin 1997/32**

(62) Numéro(s) de document de la (des) demande(s) initiale(s) en application de l'article 76 CBE:
**93402283.1 / 0 595 666**

(73) Titulaire: **INSTITUT NATIONAL DE LA SANTE ET DE LA RECHERCHE MEDICALE (INSERM)**
**75654 Paris Cédex 13 (FR)**

(72) Inventeurs:
• **Cathignol, Dominique**
**69740 Genas (FR)**
• **Lavandier, Bernard**
**42820 Ambierle (FR)**
• **Muchada, Raoul**
**69007 Lyon (FR)**

(74) Mandataire: **Portal, Gérard**
**Cabinet Beau de Loménie**
**158, rue de l'Université**
**75340 Paris Cédex 07 (FR)**

(56) Documents cités:
EP-A- 0 035 325          EP-A- 0 363 156
DE-B- 2 509 568          FR-A- 2 296 165
FR-A- 2 424 733          US-A- 4 757 822

• A.SAINZ: "Vessel area detection with c.w. ultrasound" MEDICAL AND BIOLOGICAL ENGINEERING, MBE LETTER, vol. 14, no. 2, mars 1976, STEVENHAGE,GB, pages 245-246, XP002044985
• E.WILDI: "Dynamics and Limitations of Blood/Muscle Interface Detection Using Doppler Power Returns" IEEE TRANSACTIONS ON BIO-MEDICAL ENGINEERING, vol. 27, no. 10, octobre 1980, N.Y.,US, pages 565-573, XP002044986
• R.OLSON ET J.COOKE: "A Nondestructive Ultrasonic Technique to Measure Diameter and Blood Flow in Arteries" IEEE TRANSACTIONS ON BIO-MEDICAL ENGINEERING, mars 1974, NEW YORK US, pages 168-171, XP002044987
• M.WELLS ET AL.: "Ultrasonic transesophageal measurement of Hemodynamic Parameters in Humans" ISA TRANSACTIONS, vol. 18, no. 1, 1979, PITTSBURGH US, pages 57-61, XP002044988
• M.HISTAND ET AL.: "Ultrasonic pulsed Doppler transoesophageal measurement of aortic haemodynamics in humans" ULTRASONICS, vol. 17, no. 5, septembre 1979, GUILDFORD GB, pages 215-218, XP002044989

**Description**

La présente invention concerne le domaine technique des sondes ultrasonores, au sens général, utilisées pour procéder à des mesures de vitesse et/ou de débit et elle vise en particulier le domaine des sondes ultrasonores aptes à assurer des mesures intracorporelles, en étant introduites dans le corps humain par un orifice naturel.

L'invention trouve une application particulièrement avantageuse pour la mesure du débit aortique par l'intermédiaire de la sonde introduite à l'intérieur de l'oesophage.

Le document EP-A-0 363 156 décrit un cathéter, qui est introduit à l'intérieur du conduit, dont on désire mesurer le débit volumétrique d'un liquide circulant dans celui-ci.

Par le fait même de la position du cathéter à l'intérieur du conduit, dans lequel circule le fluide, dont on veut mesurer le débit, il est fréquent que le cathéter se trouve dans une position pour laquelle les transducteurs ne sont pas dans une position diamétrale, ce qui conduit à une mesure défectueuse du diamètre et également du débit du fluide circulant dans le conduit.

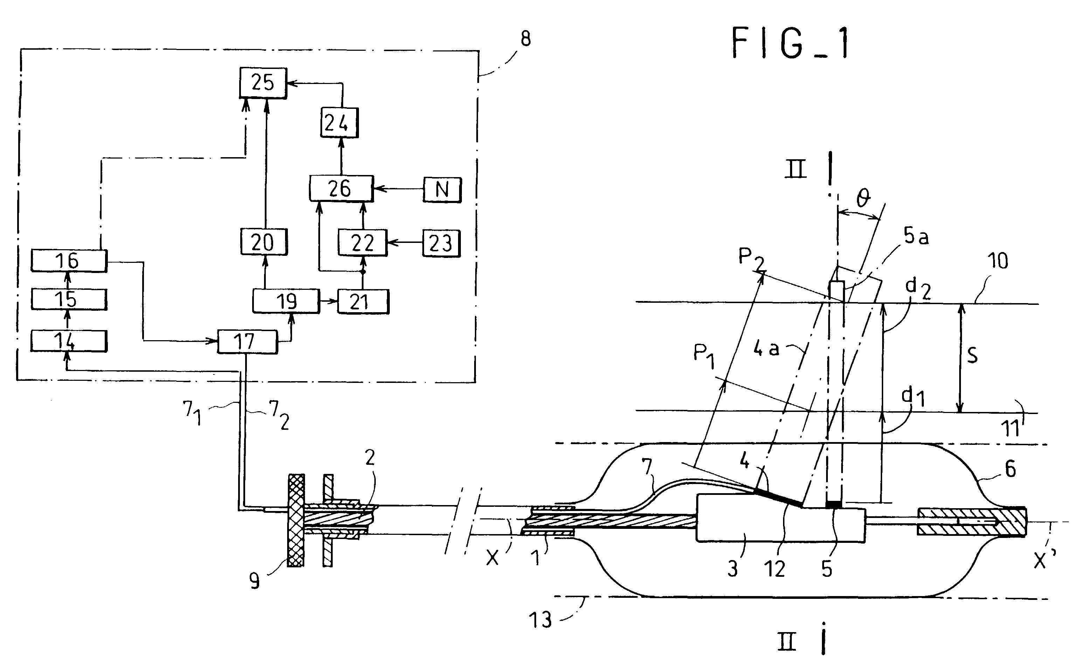

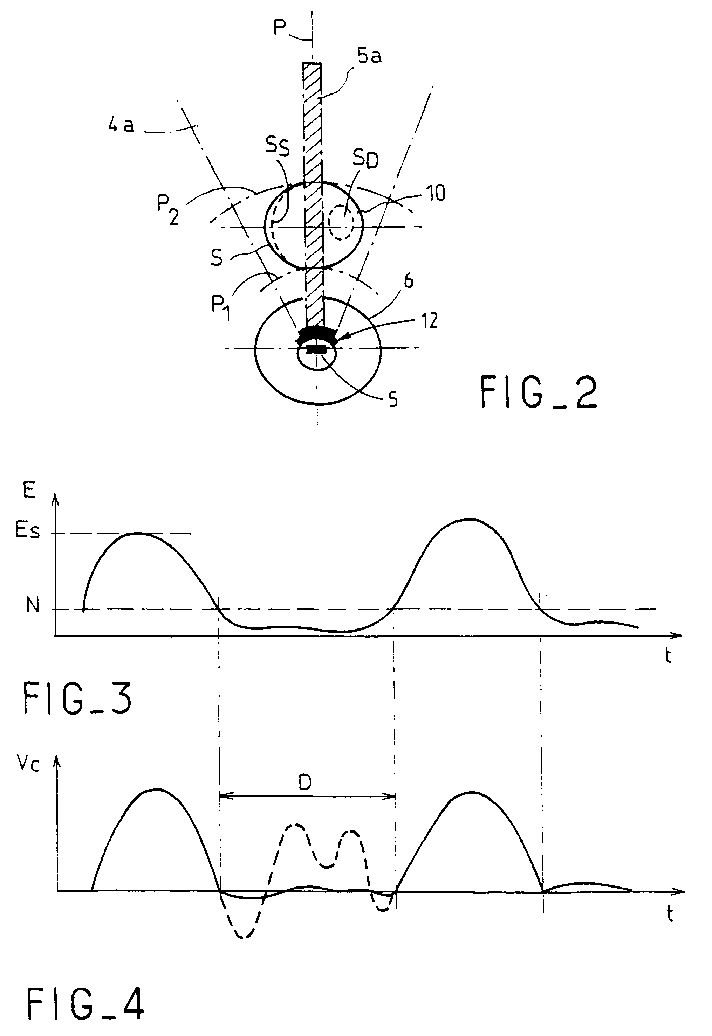

Le document FR-A-2 296 165 utilise la combinaison de deux transducteurs à effet Doppler, dont un transducteur est disposé au centre de l'autre transducteur, ces transducteurs étant obligatoirement situés dans le même plan avec la condition supplémentaire que les transducteurs soient formés par des plaques carrées (page 5, lignes 14 à 19, figures 1 et 2).

Le document FR-A-2 424 733 a proposé une sonde intracorporelle composée d'un cathéter formant une gaine souple contenant un flexible relié, par l'une de ses extrémités, à un bloc-support sur lequel est monté au moins un transducteur ultrasonore connecté, extérieurement au cathéter, à une unité de commande et de traitement. L'autre extrémité du flexible est montée solidaire d'un organe d'entraînement en rotation du bloc-support sur lui-même.

Une telle sonde permet, par effet Doppler, de déterminer la vitesse et, par suite, si le diamètre est par ailleurs connu, le débit du sang circulant à l'intérieur d'un vaisseau. Il est rappelé que le débit est égal à la section du vaisseau multiplié par la vitesse spatiale moyenne à l'intérieur du vaisseau.

Si la sonde décrite ci-dessus a permis d'effectuer un progrès important en ce qui concerne la mesure des débits aortiques, il apparaît que les mesures de débit effectuées par une telle sonde manquent de précision.

En effet, une telle sonde assure la mesure de la vitesse moyenne qui est effectuée à l'aide d'un transducteur ultrasonore dont le faisceau ne couvre pas la totalité de la surface de la section droite du vaisseau. De plus, en pratique, il apparaît difficile, voire impossible, de déterminer la bonne orientation de la sonde, afin de procéder aux mesures de débits. En effet, des positions incorrectes de la sonde peuvent donner lieu à des signaux Doppler ayant toutes les apparences du signal recherché mais conduisant à des valeurs inexactes du débit. Il s'ensuit que le faisceau ultrasonore peut couvrir aussi partiellement une zone située au dehors du vaisseau, de sorte que le dispositif de mesure prend en compte des éléments mobiles extérieurs au vaisseau, ce qui est une source d'erreur.

Il est à noter également que, dans le dispositif antérieur, le traitement effectué sur le signal Doppler ne permet pas d'exclure le mouvement des parois du vaisseau, puisque tous les signaux provenant des cibles mobiles situées tout au long du faisceau ultrasonore sont pris en compte.

Par ailleurs, au cours du cycle cardiaque, et en particulier pendant la diastole, le profil de vitesse spatiale fait apparaître des zones de vitesses nulles ou suffisamment faibles pour ne pas pouvoir être mesurées à l'aide de la sonde et du dispositif de traitement utilisé conjointement. La vitesse moyenne spatiale ainsi mesurée est erronée, puisque seules les cibles animées d'un mouvement suffisamment rapide sont prises en compte. Dans ces conditions, la mesure du débit conduit à une erreur extrêmement importante dans la mesure où la vitesse moyenne mesurée est alors multipliée par la section totale du vaisseau. Cette erreur est d'autant plus importante que la surface couverte par les veines liquidiennes immobiles ou suffisamment lentes pour ne pas être détectées par la sonde est elle-même importante par rapport à la section du vaisseau.

La présente invention vise donc à remédier aux inconvénients ci-dessus en proposant une sonde capable d'améliorer notablement la précision de la mesure de la vitesse spatiale d'une cible mobile pour une mesure précise du débit de la cible et, notamment, du débit aortique.

Un autre objet de l'invention vise à proposer une sonde apte à assurer une mesure précise de vitesse exclusivement sur toute la section de la cible mobile.

Un autre objet de l'invention vise à proposer une sonde capables d'effectuer des mesures précises de vitesse et, par suite, de débit, dans un fluide comprenant des particules en suspension, circulant dans un conduit, cette circulation pouvant être en particulier de type pulsé et ainsi présenter à certains instants une vitesse nulle ou même un flux rétrograde.

Un autre objet de l'invention vise à offrir une sonde capable d'effectuer des mesures précises de vitesse et, par suite, de débit, du sang dans l'aorte. Avantageusement, la solution doit permettre de mesurer la vitesse et donc le débit du sang dans l'aorte par l'emploi d'une sonde de type endocavitaire introduite dans l'oesophage jusqu'à être positionnée en regard de l'aorte, aussi bien pendant la diastole que la systole, ou des débits présentant des veines liquidiennes de vitesse variable notamment en raison d'un débit irrégulier ou pulsé, ce qui peut inclure une vitesse nulle ou même un flux rétrograde.

Pour atteindre les objectifs ci-dessus, la sonde de l'invention est définie dans les revendications.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

La figure 1 est une coupe en élévation d'un mode de réalisation actuellement préféré de l'invention, représenté schématiquement en position opératoire dans l'oesophage en regard de l'aorte.

La figure 2 est une vue en projection de la coupe transversale prise sensiblement selon les lignes II-II de la figure 1 et montrant un détail caractéristique de l'invention.

La figure 3 est un diagramme illustrant, dans le temps, l'énergie rétrodiffusée par la cible mobile, à savoir ici le sang circulant dans l'aorte.

La figure 4 est un exemple d'une courbe de vitesse en fonction du temps, obtenue à partir de la sonde selon l'invention selon les figures 1 et 2 avant correction (schématisé en traits discontinus) et après correction.

La figure 1 montre un exemple de réalisation d'une sonde conforme à l'invention, destinée à assurer des mesures intracorporelles de vitesse et/ou de débit. A cet effet, la sonde intracorporelle comprend un cathéter 1 formant une gaine ou un tube souple réalisé de manière connue par des matières choisies pour leurs caractéristiques de non-toxicité et de bonne tolérance pour les muqueuses. La gaine 1 contient un flexible 2 qui est relié, par l'une de ses extrémités, à au moins un bloc-support 3 sur lequel sont montés des transducteurs ultrasonores 4 et 5. D'une manière classique, cette extrémité de la sonde, qui est destinée à être introduite dans le corps, est munie d'un ballonnet 6 entourant le bloc-support 3. Les transducteurs 4, 5 sont reliés à un câble électrique 7 placé dans la gaine 1 et sortant à l'extérieur du cathéter pour être connecté à une unité 8 de commande des transducteurs et de traitement des signaux délivrés par ces derniers. L'extrémité du flexible 2, opposée de celle pourvue du bloc-support 3, est reliée à un organe de manoeuvre 9, tel qu'un bouton moleté assurant une rotation sur lui-même du flexible 2.

Selon une caractéristique de l'invention, le bloc-support 3 est aménagé pour recevoir au moins un transducteur ultrasonore 4, présentant un faisceau dit large 4a, à savoir adapté pour couvrir au moins toute la section S d'un milieu 10, tel qu'un vaisseau formant, de préférence, l'aorte et occupé par une cible mobile 11 formée, par exemple, par un flux sanguin (figure 2). Le transducteur 4 est placé sur un plan d'appui 12 ménagé dans le bloc support 3. Dans le cas où la sonde est destinée à être introduite dans l'oesophage pour permettre la mesure du débit aortique, le transducteur 4 est incliné par rapport à l'axe longitudinal x-x' commun au flexible 2 et au bloc-support 3, de manière à permettre une détection de vitesse du sang par effet Doppler. Par exemple, le transducteur 4 utilisé est le transducteur du type nature P1 60, de forme en secteur cylindrique de dimensions 4/4 mm, de rayon de courbure de 6 mm et commercialisé par la Société Quartz et Silice.

Selon l'invention, le bloc-support 3 est équipé, également, d'un transducteur ultrasonore 5 présentant un faisceau 5a considéré étroit par rapport à la section S de l'aorte 10 et par rapport au faisceau 4a. Le transducteur 5, du type à faisceau plan ou localisé, est monté sur le bloc-support 3, de manière que son faisceau 5a se trouve, de préférence, centré sur le plan symétrique P du faisceau large, passant par l'axe x-x'. Le faisceau étroit 5a est décalé d'un angle de divergence θ par rapport au faisceau large 4a et se trouve, de préférence, orienté selon une direction sensiblement perpendiculaire à l'axe x-x'.

Bien entendu, il doit être considéré que le transducteur 5 peut être fixé sur le bloc-support 3, de telle manière que son faisceau 5a se trouve dans une position relative différente mais connue par rapport à celle du faisceau large 4a. De plus, les transducteurs 4 et 5 peuvent être montés sur des blocs-supports distincts dont leurs positions relatives sont connues, en particulier par construction.

La sonde décrite dans l'exemple illustré est destinée à être introduite par un orifice naturel dans une voie naturelle, telle que l'oesophage 13 représenté en traits mixtes, puis déplacée axialement afin que les transducteurs 4 et 5 se trouvent placés en regard d'une section S de l'aorte 10. Le bloc-support 3 est alors déplacé sur lui-même par l'intermédiaire du bouton de manoeuvre 9 transmettant son effet par le flexible 2, de manière à orienter convenablement les transducteurs 4, 5 en azimut.

Afin de placer le faisceau large 4a dans une position où il couvre la section du vaisseau 10 à explorer, le transducteur 5 à faisceau étroit est relié à l'unité 8 qui comporte des moyens de mesure et de traitement des caractéristiques du signal du transducteur à faisceau étroit 5.

A cet effet, l'unité de commande et de traitement 8 comporte des moyens 14 reliés au transducteur 5 par une liaison $7_1$ et conçus pour déterminer l'amplitude des signaux reçus en écho par le transducteur à faisceau étroit 5. Les moyens de détermination 14 sont reliés à des moyens 15 conçus pour détecter les maximums de l'amplitude des signaux réfléchis. D'une manière classique, il est à considérer que l'amplitude des échos du transducteur 5 est maximum lorsque son faisceau 5a est perpendiculaire aux parois du vaisseau 10. Le bloc-support 3 est déplacé en azimut par l'intermédiaire du bouton 9, jusqu'à ce qu'il occupe une position dans laquelle l'amplitude des échos du signal du transducteur 5 apparaisse maximum. Le transducteur 4 se trouve alors, par construction, convenablement orienté, de sorte que son faisceau large 4a insonifie toute la section S de l'aorte 10. Il est ainsi possible de donner une seule et unique orientation correcte à la sonde, dans la mesure où l'amplitude des échos des parois décroît de façon importante pour un faible écart angulaire. Par ailleurs, il est à noter que le transducteur ultrasonore 4 permet de mesurer la vitesse sur toute la section du vaisseau, dans la mesure où toute cette section est insonifiée par le faisceau large 4a.

Il est à noter que, dans le cas où deux transducteurs 5 à faisceau étroit sont utilisés, il peut être envisagé de détecter, par exemple, la position dans laquelle l'amplitude des signaux des deux transducteurs 5 est égale, afin de déterminer l'orientation convenable du transducteur à faisceau large 4.

Afin d'augmenter encore la précision des mesures de vitesses par le transducteur 4, il est prévu de prendre en compte uniquement les échos du transducteur 5 provenant d'un intervalle $P_2$-$P_1$ qui circonscrit la section de l'aorte 10 en deux points extrêmes opposés. A cet effet, l'unité de commande et de traitement 8 comprend des moyens 16 conçus pour déterminer cet intervalle $P_2$-$P_1$. Les moyens 16 sont reliés aux moyens 15, afin de déterminer l'intervalle $d_2$-$d_1$ correspondant aux deux points extrêmes de l'aorte détectés à partir des maximums de l'amplitude des échos relatifs aux signaux du transducteur 5. La connaissance de l'intervalle $d_2$-$d_1$ permet de calculer la section puisqu'il est connu ou considéré que le vaisseau est de section circulaire.

Les moyens 16 déterminent, ensuite, l'intervalle $P_2$-$P_1$ à partir de l'intervalle $d_2$-$d_1$ et de l'angle de divergence θ entre les deux faisceaux, connu en particulier par construction. Ces moyens de détermination 16 pilotent des moyens de sélection 17 reliés au transducteur 4 par une liaison $7_2$. Ces moyens 17 permettent de sélectionner uniquement les échos des signaux du transducteur 4 qui sont obtenus dans une plage de temps de réponse correspondant à l'intervalle $P_2$-$P_1$. Les moyens de sélection 17 sont reliés à des moyens de traitement classique 19 assurant l'obtention d'un signal Doppler. Ces moyens de traitement 19 sont connectés à des moyens 20 connus en soi, aptes à déterminer la vitesse moyenne spatiale $V_m$ du sang traversant la section S de l'aorte 10.

L'utilisation combinée d'au moins un transducteur à faisceau large 4 et d'au moins un transducteur à faisceau étroit 5 autorise la mesure des vitesses sur toute la section de l'aorte 10 sans prendre en compte d'éléments situés à l'extérieur d'un tel vaisseau. Le champ de mesure de vitesse coïncide, ainsi, de la façon la plus étroite possible avec la section S de l'aorte.

Selon une caractéristique avantageuse de l'invention, la sonde est apte à assurer des mesures précises de vitesse moyenne spatiale qui tiennent compte de la section des veines liquidiennes animées d'une vitesse nulle ou suffisamment faible pour être considérée comme nulle par les moyens 19, 20 fonctionnant de façon classique. La sonde selon l'invention est ainsi apte à assurer des mesures de vitesse qui tiennent compte de la section effective ou réelle occupée par les hématies considérées en mouvement à l'intérieur de l'aorte.

A cet effet, l'unité 8 comprend des moyens 21 aptes à mesurer l'énergie rétrodiffusée par les particules en mouvement, à savoir les hématies dans le cas du sang. L'énergie rétrodiffusée E, qui est proportionnelle au nombre d'hématies en mouvement, est mesurée à chaque instant, afin de connaître la masse de liquide en mouvement (figure 3). Ainsi, l'énergie E du signal reçu est donnée par la formule suivante :

$$E = e.c.I.S.$$

avec c étant la concentration en particules, à savoir en hématies, e l'énergie rétrodiffusée par une particule et le produit I.S. étant le volume de mesure dans lequel se trouvent les particules en mouvement.

Les moyens de calcul de l'énergie 21, qui reçoivent le signal Doppler issu des moyens de traitement 19, déterminent à chaque instant l'amplitude ou l'énergie E rétrodiffusée par les cibles en mouvement. L'amplitude de signal Doppler est proportionnelle à la racine carrée de l'énergie rétrodiffusée. La sortie des moyens de calcul 21 est connectée à des moyens 22 conçus pour laisser passer l'énergie rétrodiffusée à un ou plusieurs instants définis, en particulier lors de la systole. Les moyens 22 sont ainsi reliés à des moyens 23 aptes à déterminer lesdits instants définis et en particulier l'instant où se produit la systole. D'une manière classique, la systole peut être détectée à partir de la vitesse maximale du sang, de l'énergie rétrodiffusée ou d'un électrocardiogramme.

Les moyens 22 délivrent donc la valeur de l'énergie rétrodiffusée $E_S$ pendant la systole. De préférence, l'énergie rétrodiffusée $E_S$, lors de la systole, est mesurée sur plusieurs cycles cardiaques, par exemple de l'ordre d'une dizaine, puis moyennée, afin de tenir compte des variations physiologiques normales.

Il doit être considéré que pendant la systole toutes les hématies sont en mouvement, de sorte que l'énergie totale rétrodiffusée $E_S$ à cet instant correspond au mouvement des cibles occupant la section totale S du vaisseau. En dehors de la systole et, notamment pendant la diastole, la surface $S_D$ couverte par les particules effectivement en mouvement est susceptible de se trouver réduite par rapport à la section complète S.

La prise en compte de l'énergie rétrodiffusée, lors de la systole $E_S$ et lors de la diastole $E_D$, permet de déterminer la surface réelle ou effective théorique $S_D$ participant au débit. Une telle surface est telle que :

$$S_D = S.(E_D/E_S) = S.K.$$

Le facteur de correction K est déterminé par des moyens de correction 24 reliés aux moyens 21, 22. D'une manière avantageuse, les moyens de correction 24 pondèrent le facteur K par un coefficient de correction pratique qui tient compte des caractéristiques techniques du transducteur 4 utilisé et des moyens 19, en particulier de la valeur minimale des vitesses détectées et de la bande passante du signal Doppler. Ces moyens de correction 24 sont connectés à des moyens 25 qui sont reliés aux moyens 20 de détermination de la vitesse moyenne spatiale. Ces moyens 25 permettent de calculer, à partir des valeurs de la vitesse moyenne spatiale et du facteur de correction K, la vitesse moyenne $V_C$ corrigée et, par suite, le débit du sang en mouvement sur la surface localisée $S_D$, à partir de la connaissance de la section du vaisseau.

La figure 4 illustre un exemple de courbe donnant la vitesse corrigée $V_C$ en fonction du temps. Cette courbe permet d'apprécier la correction effectuée à partir des vitesses brutes de mesure qui sont schématisées en traits discontinus lors de la diastole D. Le procédé selon l'invention permet d'obtenir une précision élevée sur les mesures de vitesses et, par suite, de débits du sang, puisque ces valeurs mesurées tiennent compte de la section effective participant au débit de sang.

Tel que cela apparaît plus précisément à la figure 3, le facteur de correction déterminé par les moyens 24 est appliqué uniquement lorsque l'énergie rétrodiffusée est inférieure à un seuil N donné, de nature fixe ou réglable, pour tenir compte à la fois des variations physiologiques et des variations statistiques normales du signal Doppler connues par ailleurs. Avantageusement, le seuil N est compris entre 10 et 50% de l'énergie rétrodiffusée maximale $E_S$ pendant la systole et, de préférence, de l'ordre de 25%. Une telle comparaison est effectuée par des moyens 26 interposés entre les moyens 21-22 et 24 permet de corriger à chaque instant du cycle cardiaque les valeurs de vitesse et de débit, particulièrement lors de la diastole D, comme cela apparaît plus précisément à la figure 4.

Bien entendu, les divers moyens constitutifs de l'unité 8 peuvent être réalisés d'une manière programmée ou câblée. De plus, il est à noter que les différents circuits nécessaires au fonctionnement des transducteurs 4, 5 n'ont pas été décrits plus précisément, car ils ne font pas partie de l'invention et sont connus en soi. Par ailleurs, il doit être considéré que la description qui précède concerne une sonde intracorporelle ou endocavitaire. Bien entendu, il est clair que l'objet de l'invention peut être appliqué à une sonde extracorporelle. Dans ce cas, la sonde ne comporte pas le cathéter 1 et le flexible 2. Par exemple, il peut être envisagé d'utiliser une telle sonde afin de mesurer le débit de l'aorte ascendante par voie sustemale.

Dans les dessins, la section $(S_S)$ est définie par la section occupée par les particules en suspension dans le conduit, par exemple les hématies dans l'aorte, lorsque la totalité ou sensiblement la totalité est considérée en mouvement, dans le plan de balayage de la coupe du transducteur à faisceau large 4, ladite section $(S_S)$ étant sensiblement égale à la section (S) du conduit balayé, ici l'aorte, comme représenté à la figure 2. La section partielle $(S_D)$ est la section occupée à chaque instant par les particules en suspension dans le conduit, par exemple les hématies détectées comme étant en mouvement et qui peuvent représenter une zone très localisée comme montré en figure 2.

Par rapport au facteur de correction (K), le facteur de correction (K) peut dans un exemple de réalisation être représenté par la formule suivante :

$$K = \left(\frac{E_D}{E_S}\right)^n \times k$$

dans laquelle :

K = facteur de correction
$E_D$ = énergie partielle rétrodiffusée comme précédemment définie
$E_S$ = énergie totale rétrodiffusée comme précédemment définie
n = est un nombre constituant un autre facteur de correction
k = est un coefficient de correction comme précédemment défini dépendant des caractéristiques techniques du transducteur (4) et des moyens émettant, recevant, mesurant et traitant les signaux liés au transducteur (4) à effet Doppler, incluant les moyens de mesure (19).

Lors de la mesure de la vitesse du flux de sang dans l'aorte, les valeurs suivantes ont été obtenues sur deux patients :

- avec la sonde selon l'invention, le coefficient de correction k est égal à 1, n est égal à ½ et le seuil (N) est fixé à 25 % de l'énergie maximale rétrodiffusée $(E_S)$.

Pour ces deux patients, les signaux reçus par les transducteurs de la sonde selon l'invention sont enregistrés et soumis à des calculs par ordinateur et ont donné les valeurs suivantes de mesure de vitesse :

A - Pour le patient A, ayant un diamètre d'aorte de 3 cm :

- vitesse moyenne sur 500 mesures de vitesse instantanée, non corrigée : 8,64 cm/seconde

Sur la courbe enregistrée, qui a été réalisée pendant 10 secondes, il a été observé que les mesures concernant la section partielle $(S_D)$ étaient en majorité négatives.

Puisque ce seuil était applicable, une correction de la vitesse moyenne a été réalisée en fournissant une valeur de vitesse moyenne corrigée de 9,3 cm/seconde.

Un second essai a été réalisé ultérieurement sur le même patient A et les valeurs étaient comme suit :

- vitesse moyenne sur 500 mesures de vitesse instantanée, pendant 10 secondes, non corrigée : 9,66 cm/seconde.

Il a été observé qu'en majorité, les flux localisés ($S_D$) étaient positifs et en outre que le seuil était applicable.

La vitesse moyenne corrigée était en conséquence de 9,03 cm/seconde.

B - Pour un second patient B, ayant un diamètre d'aorte de 2,4 cm, dans les mêmes conditions, les valeurs de vitesse étaient comme suit :

- vitesse moyenne, non corrigée : 18,6 cm/seconde.

Avec le patient B, les flux localisés ($S_D$) étaient en majorité positifs et le seuil était également applicable.

La valeur de vitesse corrigée était de 15,36 cm/seconde.

L'invention n'est pas limitée aux exemples décrits et représentés, car diverses modifications peuvent y être apportées sans sortir de son cadre.

**Revendications**

1. Sonde extracorporelle ou endocavitaire, pour mesurer la vitesse ou le débit d'un fluide comprenant des particules en suspension circulant dans un premier conduit (10), en particulier pour mesurer la vitesse ou le débit du sang dans l'aorte (10), ladite sonde ayant un axe longitudinal, la sonde permettant de mesurer la vitesse ou le débit du fluide pendant que la sonde est placée dans un deuxième conduit (13) extérieur dudit premier conduit (10), et comprenant une gaine (1) et au moins un bloc-support (3) pourvu de moyens de rotation (9), un élément transducteur (4) à faisceau large (4a) fixé sur ledit bloc-support (3) de manière à être orientable en rotation en direction dudit premier conduit par lesdits moyens de rotation (9), pour mesurer, par effet Doppler, la vitesse du fluide circulant dans ledit premier conduit (10) ; ainsi qu'un transducteur ultrasonore (5) à faisceau étroit (5a) monté dans ladite sonde pour présenter son faisceau étroit (5a) dans une position relative connue par rapport à celle du transducteur ultrasonore (4) à faisceau large (4a), de manière à être orientable en rotation en direction dudit premier conduit par lesdits moyens de rotation (9), lesdits moyens de rotation (9) étant prévus pour mettre en rotation le transducteur à faisceau étroit (5) autour de l'axe longitudinal de la sonde, relativement à la gaine (1), ladite sonde comprenant aussi une unité (8) de commande et de traitement comprenant :

a) des moyens (14, 15) de mesure et de traitement des caractéristiques du signal du transducteur à faisceau étroit (5a) pour indiquer la perpendicularité du faisceau étroit ultrasonore (5a) vis-à-vis de la paroi du premier conduit (10) ; ladite unité de commande (8) commandant le fonctionnement du transducteur (4) à faisceau large (4a) pour mesurer la vitesse ou le débit du fluide s'écoulant dans le premier conduit (10) quand le transducteur (5) à faisceau étroit (5a) atteint ladite perpendicularité;

b) des moyens (21) de calcul de l'énergie rétrodiffusée par les particules en mouvement à chaque instant ;

c) des moyens (22) conçus pour laisser passer l'énergie rétrodiffusée à un ou plusieurs instants définis, en particulier lors de la systole, ces moyens (22) étant eux-mêmes reliés à des moyens (23) aptes à déterminer lesdits instants définis et en particulier l'instant où se produit la systole ;

d) les moyens (22) conçus pour laisser passer l'énergie rétrodiffusée à un ou plusieurs instants définis délivrent la valeur de l'énergie rétrodiffusée totale ($E_S$) des particules en mouvement dans le premier conduit (10), lorsque toutes les particules sont en mouvement, la section occupée par toutes les particules en mouvement étant égale à $S_S$ sensiblement égale à la section S du premier conduit ;

e) les moyens (22) conçus pour laisser passer l'énergie rétrodiffusée à un ou plusieurs instants définis délivrent aussi la valeur de l'énergie rétrodiffusée partielle ($E_D$) à tout autre instant lorsque seulement une partie des particules est en mouvement, la section occupée par ces particules en mouvement étant égale à $S_D$ inférieure à $S_S$ ;

f) des moyens (19, 20) pour mesurer la vitesse moyenne spatiale lors d'une circulation des particules à un moment quelconque dans le premier conduit, et pour calculer éventuellement le débit moyen en multipliant cette vitesse moyenne spatiale par la section S du premier conduit (10), et ;

g) des moyens de correction (24) pour corriger la mesure de la vitesse moyenne spatiale ou de débit moyen par un facteur dépendant de l'énergie partielle ($E_D$) et de l'énergie totale ($E_S$).

2. Sonde selon la revendication 1, **caractérisée en ce que** l'unité (8) de commande et de traitement comprend aussi des moyens (14) reliés au transducteur à faisceau étroit (5) pour déterminer l'amplitude des signaux reçus en écho par le transducteur à faisceau étroit (5), lesdits moyens de détermination (14) sont aussi reliés à des moyens (15) conçus pour détecter les maximum de l'amplitude des signaux réfléchis, lesdits maximum correspondent à la position du faisceau (5a) du transducteur (5) perpendiculaire aux parois du premier conduit (10); l'unité de commande (8) comprenant aussi des moyens (16) conçus pour prendre uniquement les échos du transducteur à faisceau large (4) provenant d'un intervalle (P2-P1) qui circonscrit la section S du premier conduit (10).

**3.** Sonde selon la revendication 1 ou 2, **caractérisée en ce que** les moyens de correction (24) sont connectés à des moyens (25) permettant de calculer, à partir des valeurs de la vitesse moyenne spatiale et d'un facteur de correction la vitesse moyenne ($V_c$) corrigée et, par suite, le débit du fluide en mouvement sur la section $S_D$.

**4.** Sonde selon l'une des revendications 1 à 3, **caractérisée en ce qu'**elle est une sonde endocavitaire prévue pour être insérée dans l'oesophage (13), pour mesurer la vitesse et/ou le débit du sang dans l'aorte (10).

## Patentansprüche

**1.** Extrakorporale oder endokavitäre Sonde zur Messung der Geschwindigkeit oder Durchflussmenge einer Partikel in Suspension enthaltenden und in einer ersten Leitung (10) zirkulierenden Flüssigkeit, insbesondere zur Messung der Geschwindigkeit oder Durchflussmenge von Blut in der Aorta (10), welche Sonde eine Längsachse aufweist, wobei die Sonde die Messung der Geschwindigkeit oder Durchflussmenge der Flüssigkeit gestattet, während die Sonde in einer zweiten Leitung (13) außerhalb der ersten Leitung (10) platziert ist, und mit einer Hülse (1) und mindestens einem mit Rotationsmitteln (9) versehenen Trägerblock (3), einem auf dem Trägerblock (3) durch die Rotationsmittel (9) drehbar in Richtung der ersten Leitung ausrichtbar befestigten Wandlerelement (4) mit breitem Strahlenbündel (4a) zur Messung der Geschwindigkeit der in der ersten Leitung (10) zirkulierenden Flüssigkeit mittels DopplerEffekt, sowie einem in der Sonde durch die Rotationsmittel (9) drehbar in Richtung der ersten Leitung ausrichtbar befestigten Ultraschall-Wandler (5) mit schmalem Strahlenbündel (5a) zur Präsentation seines schmalen Strahlenbündels (5a) in einer bekannten Relativposition in Bezug auf jene des UltraschallWandlers (4) mit breitem Strahlenbündel (4a), wobei die Rotationsmittel (9) vorgesehen sind, um den Wandler (5) mit schmalem Strahlenbündel um die Längsachse der Sonde relativ zur Hülse (1) in Rotation zu versetzen, welche Sonde weiters eine Steuer- und Verarbeitungseinheit (8) aufweist mit:

a) Mitteln (14, 15) zur Messung und Verarbeitung der Merkmale des Signals des Wandlers mit schmalem Strahlenbündel (5a) zwecks Anzeigens der Rechtwinkeligkeit des schmalen Ultraschall- Strahlenbündels (5a) gegenüber der Wand der ersten Leitung (10), wobei die Steuereinheit (8) den Betrieb des Wandlers (4) mit breitem Strahlenbündel (4a) zur Messung der Geschwindigkeit oder Durchflussmenge der in der ersten Leitung (10) strömenden Flüssigkeit steuert, wenn der Wandler (5) mit schmalem Strahlenbündel (5a) die rechtwinkelige Position erreicht;

b) Mitteln (21) zur Berechnung der von den in Bewegung befindlichen Partikeln zu jedem Zeitpunkt rückgestrahlten Energie;

c) Mitteln (22), die so gestaltet sind, dass sie die zu einem oder mehreren bestimmten Zeitpunkten, insbesondere während der Systole, rückgestrahlte Energie durchlassen, welche Mittel (22) ihrerseits mit Mitteln (23) verbunden sind, die die bestimmten Zeitpunkte, insbesondere den Zeitpunkt der Systole, bestimmen können;

d) wobei die Mittel (22), die so gestaltet sind, dass sie die rückgestrahlte Energie zu einem oder mehreren bestimmten Zeitpunkten durchlassen, den Wert der gesamten rückgestrahlten Energie ($E_s$) der in der ersten Leitung (10) in Bewegung befindlichen Partikel liefern, wenn sämtliche Partikel in Bewegung sind, wobei der von sämtlichen Partikel in Bewegung eingenommene Querschnitt gleich $S_S$ im Wesentlichen gleich dem Querschnitt S der ersten Leitung ist;

e) wobei die Mittel (22), die so gestaltet sind, dass sie die rückgestrahlte Energie zu einem oder mehreren bestimmten Zeitpunkten durchlassen, auch den Wert der zu jedem anderen Zeitpunkt teilweise rückgestrahlten Energie ($E_D$) liefern, wenn nur ein Teil der Partikel in Bewegung ist, wobei der von diesen Partikel in Bewegung eingenommene Querschnitt gleich $S_D$ kleiner als $S_S$ ist;

f) Mitteln (19, 20) zur Messung der räumlichen Durchschnittsgeschwindigkeit während einer Zirkulation der Partikel zu irgendeinem Zeitpunkt in der ersten Leitung und gegebenenfalls Berechung der mittleren Durchflussmenge durch Multiplizieren dieser räumlichen Durchschnittsgeschwindigkeit mit dem Querschnitt S der ersten Leitung (10); und

g) Korrekturmitteln (24) zum Korrigieren der Messung der räumlichen Durchschnittsgeschwindigkeit oder mittleren Durchflussmenge um einem Faktor, der von der Teilenergie ($E_D$) und der Gesamtenergie ($E_S$) abhängt.

**2.** Sonde nach Anspruch 1, **dadurch gekennzeichnet, dass** die Steuer- und Verarbeitungseinheit (8) auch Mittel (14) aufweist, die mit dem Wandler (5) mit schmalem Strahlenbündel verbunden sind, um die Amplitude der als Echo vom Wandler (5) mit schmalem Strahlenbündel empfangenen Signale zu bestimmen, wobei die Bestimmungsmittel (14) weiters mit Mitteln (15) verbunden sind, die so ausgebildet sind, dass sie die Maxima der Amplitude der rückgestrahlten Signale detektieren, welche Maxima der Position des Strahlenbündels (5a) des Wandlers (5) senkrecht zu den Wänden der ersten Leitung (10) entsprechen, wobei die Steuereinheit (8) auch Mittel (16) aufweist, die so gestaltet sind, dass sie nur die Echos des Wandlers (4) mit breitem Strahlenbündel aufnehmen, die von einem den Querschnitt S der ersten Leitung (10) umschreibenden Abstand (P2-P1) kommen.

**3.** Sonde nach Anspruch 1 oder 2, **dadurch gekennzeichnet, dass** die Korrekturmittel (24) mit Mitteln (25) verbunden sind, die eine Berechnung der korrigierten Durchschnittsgeschwindigkeit ($V_c$) und in der Folge der Durchflussmenge der über dem Querschnitt $S_D$ in Bewegung befindlichen Flüssigkeit, ausgehend von den Werten der räumlichen Durchschnittsgeschwindigkeit und einem Korrekturfaktor, gestatten.

**4.** Sonde nach einem der Ansprüche 1 bis 3, **dadurch gekennzeichnet, dass** es sich um eine endokavitäre Sonde zur Einführung in die Speiseröhre (13) zwecks Messung der Geschwindigkeit und/oder Durchflussmenge des Bluts in der Aorta (10) handelt.

**Claims**

**1.** An extracorporal or endocavitary probe, for measuring the velocity or flow rate of a fluid containing particles in suspension flowing along a first duct (10), in particular for measuring the velocity or flow rate of blood within aorta (10), said probe having a longitudinal axis, said probe enabling to measure the velocity or flow rate of the fluid when said probe is placed in a second duct (13) outside the first duct (10), and comprising a sheath (1) and at least a support block (3) provided with rotation means (9), one broad beam ultrasound transducer (4) fixed on said support block (3) designed to be orientable by rotation into direction of said first duct by said rotation means (9), to measure, by Doppler effect, the velocity of the fluid flowing in said first duct (10); as well as a narrow beam (5a) ultrasound transducer (5) mounted in said probe to have its narrow beam (5a) in a known relative position with respect to that of the broad beam (4a) ultrasound transducer (4), designed to be orientable by rotation into direction of said first duct through said rotation means (9), said rotation means (9) be designed to rotate the narrow beam transducer (5) about the longitudinal axis of the probe, relatively to the sheath (1), said probe comprising at least a control and processing unit (8) comprising :

a) means (14, 15) for measuring and processing characteristics of the narrow beam (5a) transducer signal to indicate the perpendicular orientation of the ultrasound narrow beam (5a) relative to the wall of the first duct (10); said control unit (8) controlling the working of the broad beam (4a) transducer (4) to measure the velocity or flow rate of the fluid flowing within the first duct (10) when the narrow beam (5a) transducer (5) reaches the perpendicular orientation ;
b) calculation means (21) to calculate the backscattered energy by the particles in motion at every instant;
c) means (22) designed to pass on the backscattered energy at one or more defined instants, in particular during systole, these means (22) being themselves connected to means (23) capable of determining said defined instants and in particular the instant, when the systole occurs;
d) the means (22), designed to pass on the backscattered energy at one or more defined instants, delivered the value of the backscattered energy ($E_S$) of the particles in motion in said first duct (10), when all the particles are in motion, the cross-section occupied by all the particles in motion being equal to $S_S$ substantially equal to cross-section S of the first duct;
e) the means (22), designed to pass on the backscattered energy at one or more defined instants, also delivered the value of the partial backscattered energy ($E_D$) at every instant, when only a part of the particles is in motion, the cross-section occupied by these particles in motion being equal to $S_D$ lower to $S_S$;
f) means (19, 20) for measuring the average space velocity during a flow of said particles at any instant in the first duct, and to calculate optionally the average flow rate by multiplying said average space velocity by cross-section S of the first duct (10); and
g) correction means (24) to correct the measure of the average space velocity or average flow rate by a factor depending from the partial ($E_D$) energy and from the total energy ($E_S$).

**2.** A probe according to claim 1, **characterized in that** said control and processing unit (8) also comprises means (14) connected to the narrow beam transducer (5) to determine the amplitude of the echoes signals received by the narrow beam transducer (5), said determining means (14) being also connected to means (15) designed to detect the amplitude maximum in the reflected signals, said maxima corresponding to the position of the narrow beam (5a) of the transducer (5) perpendicular to the walls of the first duct (10); said control unit (8) comprising further means (16) designed to only take the echoes of the broad beam transducer (4) coming from a range (P2 - P1), which defines the section S of the first duct (10).

3. A probe according to claim 1 or 2, **characterized in that** said correction means (24) are connected to calculation means (25) enabling to calculate, from the values of the average space velocity and from a correction factor the corrected average velocity ($V_c$) and, then, the flow rate of the fluid in motion on the cross-section $S_D$.

4. A probe according to one of claims 1 to 3, **characterized in that** it is an endocavitary probe foreseen to be inserted into the esophagus (13), to measure the velocity and/or the flow rate of blood within aorta (10).

FIG_1

FIG_2

FIG_3

FIG_4